# EUROPÄISCHE PATENTSCHRIFT

(11) **EP 3 966 175 B1**
(45) Veröffentlichungstag und Bekanntmachung des Hinweises auf die Patenterteilung: **24.12.2025**
(21) Anmeldenummer: 20734999.4
(22) Anmeldetag: 03.07.2020
(51) Int. Cl.: A61K 6/20, C03C 3/16, A61K 6/838, A61K 8/24, A61K 8/25, A61Q 11/00, C03C 4/00

(54) **ZUSAMMENSETZUNG ZUR REMINERALISIERUNG VON ZÄHNEN**
COMPOSITION FOR THE REMINERALIZATION OF TEETH
COMPOSITION PERMETTANT LA REMINÉRALISATION DES DENTS

(30) Priorität: 04.07.2019 EP 19184355
(43) Veröffentlichungstag der Anmeldung: 16.03.2022
(73) Patentinhaber: Ferton Holding S.A., 2800 Delémont (CH)
(72) Erfinder: HEIDENAU, Frank, 91257 Pegnitz (DE)
(74) Vertreter: Isarpatent
(86) Internationale Anmeldenummer: PCT/EP2020/068810
(87) Internationale Veröffentlichungsnummer: WO 2021/001529

(56) Entgegenhaltungen:
- WO-A1-2019/034348
- US-A1- 2017 319 455
- DATABASE WPI Week 198808, Derwent World Patents Index; AN 1988-053542, XP002800528

## Beschreibung

Die vorliegende Erfindung betrifft eine Zusammensetzung zur Remineralisierung von Zähnen und deren Verwendung. Die Zusammensetzung umfasst Calciumphosphat (CaP)-Glas, und wässriges Kieselsol.

### Hintergrund der Erfindung

Calciumphosphat ist im Skelett eines der wertvollsten Materialien: Als Hauptbestandteil von Zähnen und Knochen sorgt es für Härte und Stabilität. Die Remineralisierung (Wiedereinlagerung von Mineralien) in Zähnen erhöht somit deren Härte und Widerstandskraft gegen Karies.

Im Stand der Technik sind zahlreiche Zusammensetzungen zur Remineralisierung von Zähnen bekannt.

So beschreibt beispielsweise WO 2010/041073 A1 einen Film zur Verwendung in der Mundhöhle, wobei der Film Folgendes umfasst: einen wasserlöslichen polymeren Filmbildner; und ein bioaktives Glas; wobei der Film in der Lage ist, an mindestens einem Zahn in der Mundhöhle für eine maximale Zeit von etwa 60 Minuten zu haften, bevor sich der Film auflöst oder sich im Wesentlichen auflöst, und wobei der Film in der Lage ist, eine Remineralisierung des Zahns durchzuführen. Beispielsweise umfasst der Film einen wasserlöslichen polymeren Filmbildner, ausgewählt aus Methylcellulose, Hydroxypropylmethylcellulose, Hydroxyethylcellulose, Hydroxypropylcellulose und Carboxymethylcellulose; und ein bioaktives Glas, das Siliciumdioxid, Natriumoxid, Calciumoxid und Phosphoroxid umfasst.

EP 1 343 450 bezieht sich auf einen dentalen Klebefilm zur lokalen Behandlung von Zähnen durch Remineralisation. Dieser Film besteht aus einem an den Zähnen haftenden, in Wasser löslichen oder quellbaren Trägermaterial und aus in diesem Material gespeicherten Wirkstoffen. Der Film enthält als Wirkstoff ein feinteiliges, in Wasser schwer lösliches Calciumsalz, ausgewählt aus der Gruppe der Phosphate, Fluoride, Fluorphosphate und deren Mischungen, vorzugsweise Hydroxylapatit und/oder Fluorapatit, mit einer mittleren Teilchengröße zwischen 10 und 300 nm. Darüber hinaus enthält das Trägermaterial vorzugsweise eine Proteinkomponente, vorzugsweise in Form eines Verbundmaterials, das aus einem in Wasser und Proteinkomponenten schwer löslichen Calciumsalz besteht.

EP 1 811 942 beschreibt eine Dentalzusammensetzung, umfassend: eine ethylenisch ungesättigte Verbindung mit Säurefunktionalität; eine ethylenisch ungesättigte Verbindung ohne Säurefunktionalität; und ein Calcium und Phosphor freisetzendes Glas, wobei die Säurefunktionalität eine Phosphorsäurefunktionalität umfasst.

WO 2019/068596 betrifft eine Zahnputzmittelzusammensetzung, umfassend kugelförmige, wasserfreie, amorphe Kieselgelteilchen mit einem Porenvolumen von weniger als 0,1 ml/g und einen oral verträglichen Träger.

WO 2019/034348 offenbart eine Mundpflegezusammensetzung, die ein bioaktives Glas, eine in Wasser schwach lösliche und/oder lösliche Calciumquelle, eine Phosphatquelle, und einen physiologisch akzeptablen Träger umfasst, wobei das bioaktive Glas und die leicht lösliche und/oder wasserlösliche Calciumquelle in einem Gewichtsverhältnis (a:b) von 1:3 bis 20:1 vorhanden sind; und wobei die Calciumquelle Calciumchlorid, Calciumnitrat, Calciumgluconat, Calciumglycerophosphat oder Mischungen davon ist.

JPS 63-10039 A beschreibt, dass ein Phosphat- oder Kieselsol-Einbettungsmaterial mit einer durchschnittlichen Korngröße von <=50 µm als Einbettungsmaterial für Dentalguss zum Gießen eines Calciumphosphat-Dentalmaterials verwendet wird.

US 2017/319455 A1 offenbart ein Verfahren zum Okkludieren von Dentintubuli und zur Remineralisierung von Zähnen mit einer Zahnpasta, wobei die Zahnpasta Theobromin und mindestens eines von bioaktivem Glas und Hydroxyapatit enthält.

### Zusammenfassung der Erfindung

Der vorliegenden Erfindung liegt die Aufgabe zugrunde, eine Zusammensetzung bereitzustellen, durch die Dentinoberflächen der Zähne vollständig geschützt und Unebenheiten und Defekte auf den Dentinoberflächen deutlich minimiert werden.

Die vorliegende Erfindung betrifft eine Zusammensetzung zur Remineralisierung von Zähnen umfassend oder bestehend aus:
a) Calciumphosphat (CaP)-Glas, und
b) wässrigem Kieselsol,
wobei die Zusammensetzung 30-35 Massen-% wässriges Kieselsol, 60-70 Massen-% Wasser, und 2-3 Massen-% CaP-Glas bezogen auf die Gesamtmenge der Bestandteile wässriges Kieselsol, Wasser und CaP-Glas umfasst, wobei das wässrige Kieselsol eine wässrige kolloidale Suspension nahezu kugelförmiger Polykieselsäure-Moleküle mit 10 Massen-% bis 90 Massen-% SiO₂ und den Rest Wasser ist.

Vorzugsweise ist die Zusammensetzung der vorliegenden Erfindung frei von SiO₂ als Glasbestandteil.

### Ausführliche Beschreibung der Erfindung

Wie oben beschrieben umfasst die erfindungsgemäße Zusammensetzung zur Remineralisierung von Zähnen a) Calciumphosphat (CaP)-Glas, und b) wässriges Kieselsol.

Die erfindungsgemäße Zusammensetzung dient zur Verwendung bei der Mineralisierung von Zähnen. Die Zusammensetzung wird mit einem Instrument (z.B. Wattestäbchen, Pinsel, Airflow) auf die Zahnhälse in den Zahntaschen aufgebracht. Hierbei wird eine Gelschicht (SiO₂) gebildet, die feinste Glaspartikel (Calcium-Phosphat-Glas) enthält. Diese Gelschicht härtet aus und versiegelt zunächst die frei liegenden Dentinkanäle im Zahnhals oberflächlich.

In den darauf folgenden Tagen wird das Calciumphosphatglas im Mundmilieu aufgelöst und aus dessen ionischen Bestandteilen remineralisiert in den offenen Dentinkanälen Hydroxylapatit bzw. Calciumphosphat gebildet. Dadurch werden diese dauerhaft verschlossen.

Die Empfindlichkeit der Zähne auf heiß/kalt oder süß/sauer wird dadurch vermindert und Parodontose gestoppt. Zusätzlich erfolgt eine Beruhigung der Zahntaschen in der Mundschleimhaut und im besten Falle eine Rückbildung der Parodontose geschädigten Zahntasche.

Ein Kieselsol wie hierin verwendet ist eine wässrige kolloidale Suspension nahezu kugelförmiger Polykieselsäure-Moleküle mit 10 % bis maximal 90 Massen-%, bevorzugt 15-50 Massen-% Siliciumdioxid und den Rest Wasser. Ein Beispiel für das erfindungsgemäß eingesetzte wässrige Kieselsol ist das Produkt, das unter dem Namen KÖSTROSOL^{®} vermarktet wird, hergestellt durch die Chemiewerk Bad Köstritz GmbH. Dieses Produkt kann chemisch als wässrige, kolloidale, schwach alkalische Kieselsäuredispersion definiert werden. Die Bestandteile der Zusammensetzung sind wie folgt:

| Komponenten | CAS-Nr. | EINECS-Nr. | Massen-% |
|---|---|---|---|
| amorphe | 7631- | 231- | 15 - 50 |
| Kieselsäure | 86-9 | 545-4 | |
| Wasser | 7732- | 231- | 44,5 - 84,5 |
| 18-5 | | | 791-2 |

Das CaP-Glas wird vorzugsweise gemahlen und gesiebt, und weißt eine Partikelgröße < 100 µm auf. In einer bevorzugten Ausführungsform weist die Partikelgröße des CaP-Glases einen D₅₀ < 35, bevorzugt < 20, oder < 10 µm und/oder einen D₉₀ < 90, bevorzugt < 20 µm auf.

Die Bestimmung der Partikelgröße erfolgt vorzugsweise über eine Siebanalyse (beispielsweise Siebturm), wobei die Partikelgrößenverteilung auf die Masse bezogen ist. Dies bedeutet, dass beispielsweise bei einem D₅₀ = 10 µm 50 % Masseanteil der Partikel in einer gegebenen Probe eine Größe < 10 µm und 50 % Masseanteil der Partikel eine Größe > 10 µm aufweisen. Analog wäre bei einem D₉₀ = 20 µm der Masseanteil der Partikel in einer gegebenen Probe mit einer Größe < 20 µm bei 90 % und der Masseanteil der Partikel einer Größe > 90 µm bei 10 %.

Die Siebanalyse wird beispielsweise in der Deutschen Norm DIN 66165 beschrieben. Die Norm besteht aus zwei Teilen; DIN 66165-1 definiert die Grundlagen, DIN 66165-2 die Durchführung der Siebanalyse.

Die erfindungsgemäße Zusammensetzung enthält weiterhin zusätzlich vorzugsweise vollentsalztes Wasser und/oder verdünnte Salzsäure. Das vollentsalzte Wasser wird vorzugsweise zur Sterilisation abgekocht. Damit sind die bevorzugten Bestandteile der erfindungsgemäßen Zusammensetzung Calciumphosphat (CaP)-Glas, wässriges Kieselsol, vollentsalztes Wasser und verdünnte Salzsäure (zur pH-Einstellung). Beispielsweise kann die verdünnte Salzsäure aus 1,62 g 35%ige Salzsäure auf 100 g VE-Wasser hergestellt werden. Der bevorzugte pH-Wert Zusammensetzung beträgt ungefähr pH 6.6-6.7

In einer weiteren Ausführungsform enthält die erfindungsgemäße Zusammensetzung weiterhin Chlorhexidindigluconat und/oder verdünnte Salzsäure.

Chlorhexidindigluconat wird allgemein zur Vorbeugung und Behandlung von Infektionskrankheiten, zum Beispiel bei kleinen Wunden und Verletzungen, bei leichten Verbrennungen sowie bei einer Zahnfleischentzündung und anderen entzündlichen und infektiösen Erkrankungen des Mund- und Rachenraums verwendet. Der eigentliche wirksame Bestandteil ist Chlorhexidin, ein antiseptischer Wirkstoff aus der Gruppe der Desinfektionsmittel, der zur Vorbeugung und Behandlung von Infektionskrankheiten und für die Mundhygiene eingesetzt wird. Das chlorierte Biguanid-Derivat ist vor allem gegen Bakterien wirksam. Die Effekte beruhen auf einer Störung der Funktion der Zellmembran.

Chlorhexidindigluconat wird der Zusammensetzung in pharmazeutisch üblichen Dosierungen zugesetzt (0,1 - 0,2 % w/w).

Das erfindungsgemäß eingesetzte Calciumphosphat (CaP)-Glas wird vorzugsweise aus äquimolaren Mengen CaCO₃ und P₂O₅ hergestellt. Ein genaues Herstellungsbeispiel für das erfindungsgemäße Calciumphosphat-Glas findet sich in den nachfolgenden Ausführungsbeispielen.

Die erfindungsgemäße Zusammensetzung zur Remineralisierung von Zähnen umfasst folgende Bestandteile: SiO₂-Sol 30-35 % w/w, Wasser 60-70 % w/w, und CaP-Glas 2-3 % w/w bezogen auf die Gesamtmenge der Bestandteile SiO₂-Sol, Wasser und CaP-Glas.

Hinzu kommt optional eine geringe Menge verdünnter HCl zur pH-Einstellung bzw. Chlorhexidindigluconat.

Der pH der Zusammensetzung ist vorzugsweise ungefähr pH 6.6-6.7

Beispielsweise umfasst die Zusammensetzung folgende Mengenanteile der Bestandteile in der Trockensubstanz:
- Massenanteil Ca in der Trockensubstanz: 20,4 Massen-%
- Massenanteil P in der Trockensubstanz: 31,2 Massen-%
- Massenanteil Si in der flüssigen Komponente (Köstrosol): 4,67 Massen-%
- Massenanteil Si in der Gesamtmischung: 4,55 Massen-%

Gemäß eines weiteren Aspekts der vorliegenden Erfindung wird die oben geschilderte Zusammensetzung zur Remineralisierung von Zähnen verwendet. Wie bereits oben geschildert wird die Zusammensetzung mit einem Instrument auf die Zahnhälse in den Zahntaschen aufgebracht wird. Hierbei wird eine Gelschicht (SiO₂) gebildet, die feinste Glaspartikel (Calcium-Phosphat-Glas) enthält. Diese Gelschicht härtet aus und versiegelt zunächst die frei liegenden Dentinkanäle im Zahnhals oberflächlich. In den darauf folgenden Tagen wird das Calciumphosphatglas im Mundmilieu aufgelöst und aus dessen ionischen Bestandteilen remineralisiert in den offenen Dentinkanälen Hydroxylapatit bzw. Calciumphosphat gebildet.

### Kurze Beschreibung der Abbildungen

Abbildung 1: links) Bestückung des Ofens; rechts) Programmierung des Ofens.
Abbildung 2: REM-Aufnahmen (Vergrößerung 2500) von behandelten Dentinoberflächen nach 0d, 1d, 7d und 14d.
Abbildung 3: Dentinoberflächen mit senkrecht verlaufenden Dentinkanälen vor (0d) und nach (14d) der Behandlung mit der erfindungsgemäßen Zusammensetzung.
Abbildung 4: Topographische REM-Aufnahme der unbehandelten (0d) und behandelten (14d) Dentinoberfläche.
Abbildung 5: Querschnitt durch Dentintubuli einer mit der erfindungsgemäßen Zusammensetzung behandelten Dentinoberfläche nach 14d (im Bild oben).

### Beispiele

### 1 Herstellung des Calciumphosphatglases

### 1.1 Herstellung der Glas-Rohmischung

Zur Herstellung einer Tiegelfüllung des Calciumphosphatglases werden 101.09 g CaCO₃ (1,00 mol) und 141.94 g P₂O₅ (1,00 mol) benötigt.

25,27 g CaCO₃ (0,250 mol) abgewogen und in einen Achatmörser gegeben. Mit einem Keramikspatel werden dann zügig 35,48 g P₂O₅ (0,250 mol; Achtung, stark hygroskopisch!) abgewogen und dem CaCO₃ in dem Achatmörser hinzugefügt.

Die Mischung wird 2 min innig verrieben. Dann darf die Mischung 5 min ruhen und wird anschließend nochmals 2 min verrieben.

Die Mischung wird in den Aluminiumoxidtiegel gefüllt und eine weitere Portion aus 25,27 g CaCO₃ und 35,48 g P₂O₅ wie oben beschrieben präpariert, usw. Aufgrund des Fassungsvermögens des Achatmörsers dürfen keine größeren Mengen auf einmal verrieben werden um eine gute Durchmischung zu gewährleisten.

### 1.2 Vorbereitung des Brennofens (Glasschmelzen)

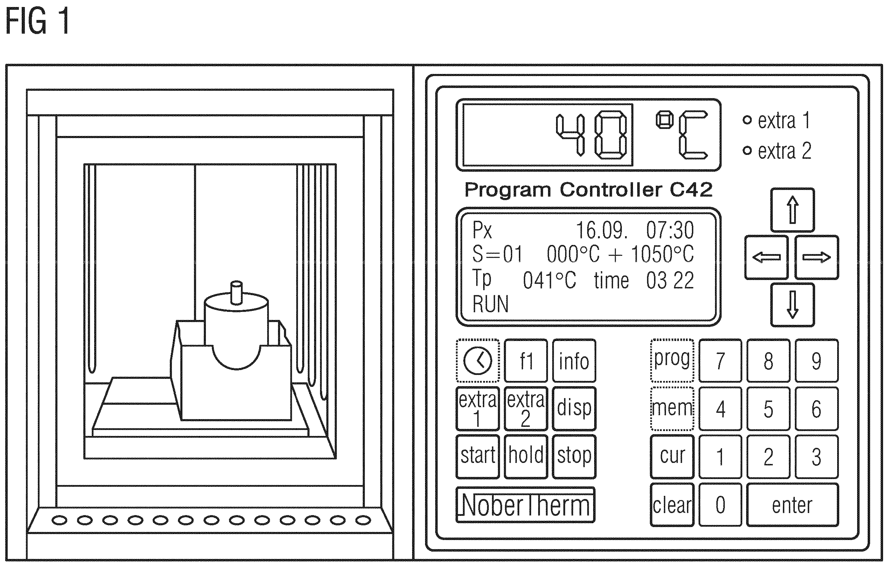

Der Ofen wird wie in Abb. 1 dargestellt bestückt. Der Ofen wird verschlossen und das Sinterprogramm mit einer Aufheizrate von 0°C-1050°C: 300K/h gewählt.

Siehe Abbildung 1: links) Bestückung des Ofens; rechts) Programmierung des Ofens.

### 1.3 Glasgießen

Es ist ein hitzebeständiger Eimer mit kaltem Leitungswasser (kein VE-Wasser, um Auslaugung des Glases zu vermeiden!) bereitzustellen.

Der Tiegel wird mit der Zange gefasst (sicheren Griff überprüfen), bis kurz oberhalb des Eimers (Spritzer vermeiden) geführt und die Glasschmelze in das kalte Wasser gegossen. Der Vorgang ist sicher aber zügig durchzuführen, um ein Abkühlen der Glasschmelze im Tiegel zu vermeiden.

Im Anschluss ist der Tiegel zurück in die Keramikschale im Ofen zu stellen, der Ofen zu verschließen und frei abkühlen zu lassen.

Das erstarrte Glas ist baldmöglichst aus dem Wasser zu entnehmen und im Trockenschrank bei 40 °C, 16 h zu trocknen. Danach kann es gemahlen werden.

### 1.4 Glasmahlen

Das erstarrte Glas ist grob zu zerkleinern und anschließend auf zwei Aluminiumoxid-Mahlbecher zu verteilen. Es werden Aluminiumoxid-Mahlkugeln (3 mittlere Kugeln d = 1,5 cm, 4 kleine Kugeln, d = 1 cm) hinzugefügt und das Glas für 4 h in der Planetenkugelmühle bei 200 UpM gemahlen. Um sicherzustellen, dass keine größeren Partikel zurückbleiben (es können Glassplitter und größere Körner auftreten!), wird das gemahlene Glas mit einem Sieb (200 µm) im Siebturm gesiebt. Zur Bestimmung der mittleren Partikelgröße wird eine Probe entnommen und mittels Laser-Granulometer vermessen (D₅₀ < 25 µm).

### 2 Herstellung der Mischungskomponenten

### 2.1 Chemikalien

- Köstrosol^{®} 0830 (wässriges Kieselsol, siehe "Datenblatt Köstrosol 830")
- vollentsalztes Wasser (VE-Wasser), wird zur Sterilisation abgekocht
- verdünnte Salzsäure (1,62 g 35%ige Salzsäure auf 100g VE-Wasser)
- Calciumphosphat-Glas (gemahlen und gesiebt; Partikelgröße < 100µm, siehe Kap. 1)
- Chlorhexidindigluconat 20%ig (optional)

### 2.2 Geräte

- Analysenwaage Sartorius CP324S
- Brand Transferpette S (Volumen 500 - 5000 µl)
- Brand Transferpette S (Volumen 100 -1000 µl)
- pH-Meter Portamess 911 pH (Fa. Knick)
- 150 ml Bechergläser (zur Herstellung der flüssigen Komponente)
- 1 kleines Rollrandglas (für die Einwaage von Calciumphosphat-Glas)
- 12 Rollrandgläser (50 mm x 20 mm, 15ml Inhalt) für die Anwendungseinheiten
- 12 passende Deckel
- 12 Magnetrührstäbchen (10 x 6 mm)
- Multipointrührer (Fa. VarioMag)

Bechergläser, Rollrandgläser und Magnetrührstäbchen werden zur Sterilisation in vollentsalztem Wasser (VE-Wasser) abgekocht. Die Deckel werden mit Bacillol^{®} (Reinigungsmittel auf der Basis von 2-Propanol, 1-Propanol und Ethanol) desinfiziert.

### 2.3 Herstellung der flüssigen Komponente

Es werden 26,8 g Köstrosol^{®} und 53,2 g abgekochtes Wasser eingewogen. Anschließend werden beide Komponenten zusammengegeben und ca. 15 Minuten gerührt.

### 2.4 Herstellung der Suspensionen

Pro Anwendungseinheit werden 5 g der flüssigen Komponente eingesetzt. Die Einwaage erfolgt auf der Analysenwaage Sartorius CP324S. Es werden jeweils 4710 µl der flüssigen Komponente (Dichte: 1,06 g/ml) mit der Brand Transferpette S (Volumen 500 - 5000 µl) in die 12 Rollrandgläser pipettiert. Anschließend werden jeweils 0,125 g der festen Komponente (Calciumphosphat-Glas) in einem kleinen Rollrandglas auf der Analysenwaage Sartorius CP324S eingewogen und in die 12 Rollrandgläser mit der flüssigen Komponente überführt.

Durch Vereinigung von flüssiger und fester Komponente sind Suspensionen entstanden. Die Rollrandgläser werden mit den zugehörigen Deckeln verschlossen. Die Proben werden anschließend ca. 1h auf dem Multipointrührer (Fa. VarioMag) bei 700-750 RPM gerührt.

### 2.5 pH-Wert-Messung und HCI-Zugabe

Nach 1h Rührzeit werden mit der Brand Transferpette S (Volumen 100 -1000 µl) jeweils 300 µl verdünnte Salzsäure hinzugefügt. Dann werden die 12 Rollrandgläser wieder verschlossen. Die Proben werden über Nacht gerührt.

### 2.6 Zugabe von Chlorhexidindigluconat (20%ig)

Abschließend wird Chlorhexidindigluconat (20%) hinzugefügt. Die Einwaage erfolgt auf der Analysenwaage Sartorius CP324S. Es werden jeweils 50 µl Chlorhexidindigluconat (20%ig) mit der Brand Transferpette S (Volumen 100 - 1000 µl) in die 12 Proben überführt. Die einzelnen Einwaagen werden dokumentiert. Nach der Chlorhexidindigluconat-Zugabe werden die Proben wieder verschlossen und noch 5 min weitergerührt. Es bildet sich ein flockiger Niederschlag. Nach längerem Stehen (ca. 2 h) werden die Proben fest.

### 3 Verschluss von Dentinkanälen im in vitro Experiment

### 3.1 Probenvorbereitung

Zahnproben wurden quer zu den Dentinkanälen poliert. Remineralisierungs-Suspension wurde mit Wattestäbchen auf die gereinigten und getrockneten Proben aufgetragen. Die Einwirkzeit der Suspension auf dem Zahn (Dentin) betrug 10 Minuten. Danach erfolgte eine Auslagerung der Proben für definierte Zeiten (0d, 1d, 7d, 14d) in Kochsalzlösung (Medium wurde täglich gewechselt). Dieser Aufbau diente der Simulation des Milieus im Mundraum. Zu den Beaobachtungszeiten wurden die Proben entnommen, getrocknet und mittels Rasterelektronenmikroskopie (REM) analysiert.

### 3.2 Versuchsergebnisse

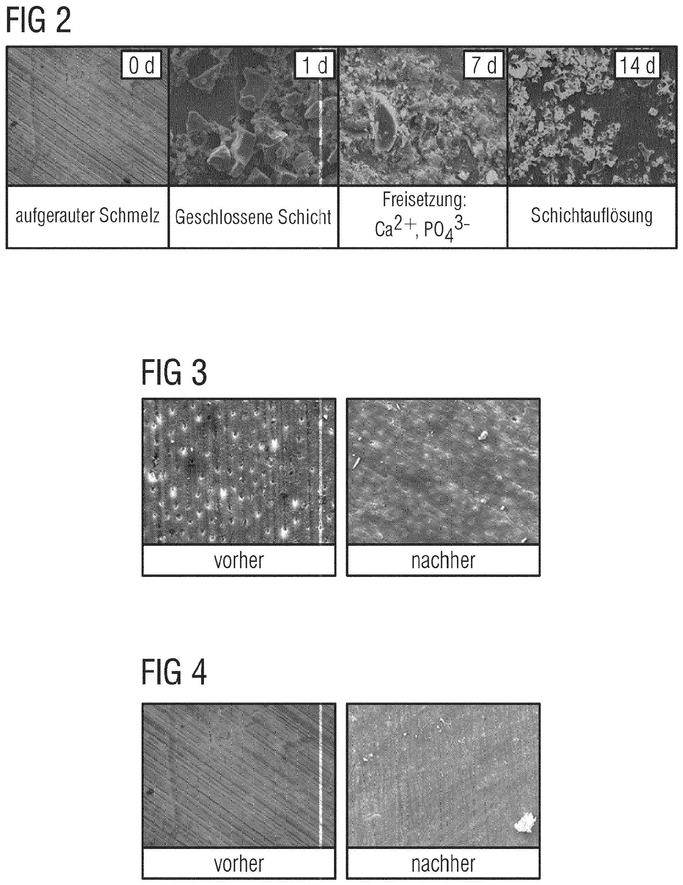

Die REM-Bilder in Abb. 2 zeigen die behandelten Dentinoberflächen an den verschiedenen Beobachtungszeitpunkten (0d, 1d, 7d, 14d).

### Siehe Abbildung 2: REM-Aufnahmen (Vergrößerung 2500) von behandelten Dentinoberflächen nach 0d, 1d, 7d und 14d.

Zu Zeitpunkt 0d ist nur aufgerauter Schmelz vorhanden. Anschließend wird die im Anwendungsbeispiel genannte Bioglas-basierte Remineralisierungspaste aufgetragen. Nach 1d ist noch eine geschlossene Schicht erkennbar. Nach 7d beginnt die Auflösung der Beschichtung und die Freisetzung von Calcium- und Phosphationen beginnt. Diese Ionen remineralisieren zu Calciumphosphat und verschließen unter der Beschichtung durch Überschreiten des Löslichkeitsproduktes von Calcium und Phosphat die Dentinkanäle.

Nach vollständiger Auflösung der Beschichtung können zwei Effekte beobachtet werden (Abb. 3, 4).

Abb. 3 zeigt Dentinoberflächen mit senkrecht verlaufenden Dentinkanälen vor (0d) und nach (14d) der Behandlung mit der erfindungsgemäßen Zusammensetzung. In Abb. 3 ist zu erkennen, dass im Vergleich zu der unbehandelten Oberfläche die Dentinkanäle vollständig geschlossen sind.

In Abb. 4 ist ersichtlich, dass die Unebenheiten und Defekte auf der Dentinoberfläche durch die Behandlung deutlich minimiert wurden. Abb. 4 zeigt eine topographische REM-Aufnahme der unbehandelten (0d) und behandelten (14d) Dentinoberfläche.

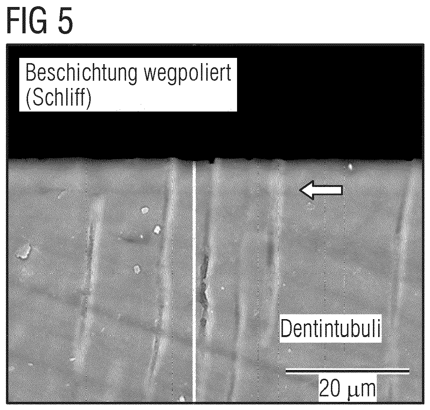

Fertigt man einen Querschliff zu den Dentinkanälen an (Abb. 5), so ist deutlich deren Verschluss durch die Behandlung mit der Bioglas-basierten Remineralisierungspaste erkennbar.

## Patentansprüche

1. Zusammensetzung zur Remineralisierung von Zähnen umfassend:
a) Calciumphosphat (CaP)-Glas, und
b) wässriges Kieselsol,
wobei die Zusammensetzung 30-35 Massen-% wässriges Kieselsol, 60-70 Massen-% Wasser, und 2-3 Massen-% CaP-Glas bezogen auf die Gesamtmenge der Bestandteile wässriges Kieselsol, Wasser und CaP-Glas umfasst,
wobei das wässrige Kieselsol eine wässrige kolloidale Suspension nahezu kugelförmiger Polykieselsäure-Moleküle mit 10 Massen-% bis 90 Massen-% SiO₂ und den Rest Wasser ist.

2. Zusammensetzung nach Anspruch 1, wobei das CaP-Glas gemahlen und gesiebt ist, und eine Partikelgröße < 100 µm aufweist.

3. Zusammensetzung nach Anspruch 2, wobei die Partikelgröße des CaP-Glases einen D₅₀ < 35 µm und/oder einen D₉₀ < 90 µm aufweist.

4. Zusammensetzung nach einem oder mehreren der vorhergehenden Ansprüche, die weiterhin vollentsalztes Wasser und verdünnte Salzsäure enthält.

5. Zusammensetzung nach einem oder mehreren der vorhergehenden Ansprüche, die weiterhin Chlorhexidindigluconat und/oder verdünnte Salzsäure enthält.

6. Zusammensetzung nach einem oder mehreren der vorhergehenden Ansprüche, wobei das Calciumphosphat-Glas aus äquimolaren Mengen CaCO₃ und P₂O₅ hergestellt ist.

7. Verwendung der Zusammensetzung nach einem oder mehreren der vorhergehenden Ansprüche zur Remineralisierung von Zähnen.

## Claims

1. Composition for the remineralisation of teeth comprising:
a) calcium phosphate (CaP) glass, and
b) aqueous silica sol,
wherein the composition comprises 30-35% by mass aqueous silica sol, 60-70% by mass water, and 2-3% by mass CaP glass relative to the overall quantity of the components aqueous silica sol, water and CaP glass,
wherein the aqueous silica sol is an aqueous colloidal suspension of almost spherical polysilicic acid molecules with 10% by mass to 90% by mass SiC₂ and the remainder water.

2. Composition according to claim 1, wherein the CaP glass is ground and sieved, and has a particle size < 100 µm.

3. Composition according to claim 2, wherein the particle size of the CaP glass has a D₅₀ < 35 µm and/or a D₉₀ < 90 µm.

4. Composition according to one or more of the preceding claims, which further contains fully demineralised water and dilute hydrochloric acid.

5. Composition according to one or more of the preceding claims, which further contains chlorhexidine digluconate and/or dilute hydrochloric acid.

6. Composition according to one or more of the preceding claims, wherein the calcium phosphate glass is produced from equimolar quantities of CaCO₃ and P₂O₅.

7. Use of the composition according to one or more of the preceding claims for the remineralisation of teeth.

## Revendications

1. Composition pour la reminéralisation de dents comprenant :
a) du verre de phosphate de calcium (CaP), et
b) du sol de silice aqueux,
la composition comprenant 30 à 35 % en masse de sol de silice aqueux, 60 à 70 % en masse d'eau et 2 à 3 % en masse de verre de CaP par rapport à la quantité totale des composants sol de silice aqueux, eau et verre de CaP,
le sol de silice aqueux étant une suspension colloïdale aqueuse de molécules d'acide polysilicique quasi sphériques avec 10 % à 90 % en masse de SiO₂, le reste étant de l'eau.

2. Composition selon la revendication 1, dans laquelle le verre de CaP est broyé et tamisé et présente une taille de particules < 100 µm.

3. Composition selon la revendication 2, dans laquelle la taille de particules du verre de CaP présente un D₅₀ < 35 µm et/ou un D₉₀ < 90 µm.

4. Composition selon l'une ou plusieurs des revendications précédentes, qui contient en outre de l'eau entièrement déminéralisée et de l'acide chlorhydrique dilué.

5. Composition selon l'une ou plusieurs des revendications précédentes, qui contient en outre du digluconate de chlorhexidine et/ou de l'acide chlorhydrique dilué.

6. Composition selon l'une ou plusieurs des revendications précédentes, dans laquelle le verre de phosphate de calcium est préparé à partir de quantités équimolaires de CaCO3 et de P₂O₅.

7. Utilisation de la composition selon l'une ou plusieurs des revendications précédentes pour la reminéralisation de dents.
